# EUROPEAN PATENT APPLICATION

(11) **EP 3 751 878 A1**
(43) Date of publication of application: **16.12.2020**
(21) Application number: 19179925.3
(22) Date of filing: 13.06.2019
(51) Int. Cl.: H04W 12/06, H04L 29/06, G06F 21/35, A24F 47/00, A61M 11/04, A61M 15/06

(54) **A METHOD FOR MANAGING A SYSTEM WITH A SMOKING SUBSTITUTE DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A user authentication system comprising a smoking substitute device comprising an authentication component and an authentication requesting device configured to communicate an authentication request to the smoking substitute device to authorise a user action. The smoking substitute device is configured to receive the authentication request and, in response to the received authentication request, communicate a identifier to the authentication requesting device. The authentication requesting device is capable of authenticating the user using the identifier to authorise performance of the user action.

## Description

### TECHNICAL FIELD

The present invention relates to smoking substitute devices. In particular, although not exclusively, it relates to the use of smoking substitute devices in communication with other devices.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful byproducts. There have been proposed various smoking substitute devices in order to avoid the smoking of tobacco.

Such smoking substitute devices can form part of nicotine replacement therapies aimed at people who wish to stop smoking and overcome a dependence on nicotine.

Smoking substitute devices may comprise electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute devices are intended to provide a substitute for the rituals of smoking, whilst providing the user with a similar experience and satisfaction to those experienced with traditional smoking and tobacco products. Some smoking substitute systems use smoking substitute articles (also referred to as a "consumables") that are designed to resemble a traditional cigarette and are cylindrical in form with a mouthpiece at one end.

The popularity and use of smoking substitute devices has grown rapidly in the past few years. Although originally marketed as an aid to assist habitual smokers wishing to quit tobacco smoking, consumers are increasingly viewing smoking substitute devices as desirable lifestyle accessories. Some smoking substitute devices are designed to resemble a traditional cigarette and are cylindrical in form with a mouthpiece at one end. Other smoking substitute devices do not generally resemble a cigarette (for example, the smoking substitute device may have a generally box-like form).

There are a number of different categories of smoking substitute devices, each utilising a different smoking substitute approach. A smoking substitute approach corresponds to the manner in which the substitute system operates for a user.

One approach for a smoking substitute device is the so-called "vaping" approach, in which a vapourisable liquid, typically referred to (and referred to herein) as "e-liquid", is heated by a heating device to produce an aerosol vapour which is inhaled by a user. An e-liquid typically includes a base liquid as well as nicotine and/or flavourings. The resulting vapour therefore typically contains nicotine and/or flavourings. The base liquid may include propylene glycol and/or vegetable glycerin.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices which typically have a sealed tank and heating element which is pre-filled with e-liquid and is not intended to be refilled by an end user. One subset of closed system vaping smoking substitute devices include a main body which includes the power source, wherein the main body is configured to be physically and electrically coupled to a consumable including the tank and the heating element. In this way, when the tank of a consumable has been emptied, the main body can be reused by connecting it to a new consumable. Another subset of closed system vaping smoking substitute devices are completely disposable, and intended for one-use only.

There are also "open system" vaping smoking substitute devices which typically have a tank that is configured to be refilled by a user, so the device can be used multiple times.

An example vaping smoking substitute device is the myblu™ e-cigarette. The myblu™ e-cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick which is partially immersed in the e-liquid. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

Another example vaping smoking substitute device is the blu PRO™ e-cigarette. The blu PRO™ e-cigarette is an open system device which includes a main body, a (refillable) tank, and a mouthpiece.

The main body and tank are physically and electrically coupled together by screwing one to the other. The mouthpiece and refillable tank are physically coupled together by screwing one of the other, and detaching the mouthpiece from the refillable tank allows the tank to be refilled with e-liquid. The device is activated by a button on the main body. When the device is activated, electrical energy is supplied from the power source to a heating device, which heats e-liquid from the tank to produce a vapour which is inhaled by a user through the mouthpiece.

Another approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

There may be a need for improved design of smoking substitute systems, in particular HT smoking substitute systems, to enhance the user experience and improve the function of the HT smoking substitute system.

An example of the HT approach is the IQOS™ smoking substitute device from Philip Morris Ltd. The IQOS™ smoking substitute device uses a consumable, including reconstituted tobacco located in a wrapper. The consumable includes a holder incorporating a mouthpiece. The consumable may be inserted into a main body that includes a heating device. The heating device has a thermally conductive heating knife which penetrates the reconstituted tobacco of the consumable, when the consumable is inserted into the heating device. Activation of the heating device heats the heating element (in this case a heating knife), which, in turn, heats the tobacco in the consumable. The heating of the tobacco causes it to release nicotine vapour and flavourings which may be drawn through the mouthpiece by the user through inhalation.

A second example of the HT approach is the device known as "Glo"™ from British American Tobacco p.l.c. Glo™ comprises a relatively thin consumable. The consumable includes leaf tobacco which is heated by a heating device located in a main body. When the consumable is placed in the main body, the tobacco is surrounded by a heating element of the heating device. Activation of the heating device heats the heating element, which, in turn, heats the tobacco in the consumable. The heating of the tobacco causes it to release nicotine vapour and flavourings which may be drawn through the consumable by the user through inhalation. The tobacco, when heated by the heating device, is configured to produce vapour when heated rather than when burned (as in a smoking apparatus, e.g. a cigarette). The tobacco may contain high levels of aerosol formers (carrier), such as vegetable glycerine ("VG") or propylene glycol ("PG").

The present inventor(s) have observed that most smoking substitute devices currently on the market are configured to operate in isolation of other devices, which limits the functions the smoking substitute devices can perform.

The present inventor(s) have observed that there is an increasing demand and appreciation, amongst users, for smoking substitute devices having functionality beyond their core functionality.

The present invention has been devised in light of the above considerations.

### Summary of the Invention

At its most general, the present invention provides a system, method, computer implemented method, computer program and devices, which enable a smoking substitute device to be used to authenticate a user in a secure and user-controllable manner. This enhances the usefulness of the smoking substitute device to the user and has the potential to reduce the number of separate devices that a user needs to own and use, in order to carry out the activities as part of his or her day-to-day life.

The smoking substitute device, once initially configured with suitable user-authentication data, may store that data (e.g. in an encrypted form) and provide it to an authentication requesting device, without requiring input from any additional device or network-based entity. Therefore, the smoking substitute device can be used as a standalone means of authenticating the identity of a user. A smoking substitute device configured in this way can provide user authentication to authorise certain user actions including: commercial transactions, which may require age or identity verification in addition to the provision of payment; entry to locations exclusively for users of the device, or for users who are members of a particular group; and signing for, or otherwise confirming safe receipt of, a delivery. It does this by receiving and responding to a user authentication request from a device that requires authentication, such as a payment terminal, electronic door release, or delivery recordal device, and wherein the device that requires authentication will only permit performance of the user action if the response from the smoking substitute device meets one or more predetermined criteria.

According to a first aspect of the present invention, there is provided a user authentication system comprising: a smoking substitute device comprising an authentication component; and an authentication requesting device configured to communicate an authentication request to the smoking substitute device to authorise a user action. The smoking substitute device is configured to: receive the authentication request; and in response to the received authentication request, communicate a identifier to the authentication requesting device. The authentication requesting device is capable of authenticating the user using the identifier to authorise performance of the user action.

Before the authentication requesting device communicates an authentication request to the smoking substitute device to authorise a user action, the smoking substitute device (or the user of the smoking substitute device) may make a request to perform the user action and/or may attempt to perform the user action and/or may make preparations to perform the user action. For example, when the authentication requesting device and the smoking substitute device communicate with one another via a proximity-based technology (discussed further below), the user may bring his or her smoking substitute device close to the authentication requesting device, to prompt it to issue the authentication request to the smoking substitute device. For example, when the user action is to purchase goods from a retailer, the user may proceed with the normal process of selecting the goods and having the goods processed at the 'checkout' or other payment point (which may be online or in person), and then, before payment can occur, the authentication requesting device may issue its authentication request to the smoking substitute device. The user action may thus be a commercial transaction and the authentication request may comprise a request to authenticate the user in order to authorise the performance of a commercial transaction.

The authentication requesting device may be configured to authenticate the user to authorise performance of the user action if the received identifier indicates that a user fulfils at least one predetermined criterion. For example, the predetermined criterion may be that the user's identity, or code, or membership number, or other identifying details, must be pre-stored on a database or other list. For example, the identifier may comprise information indicative of an age of the user and the predetermined criterion may be that the age of the user must equal or exceed a predetermined age limit. For example, the user may have to be at least 16 years of age, or at least 18 years of age, or at least 21 years of age, in order to carry out some user actions. Alternatively, there may be some user actions that are only permissible for users whose age is within a predetermined age range.

The identifier may comprise user identification information that can be verified, or for which evidence has previously been provided. For example, the identifier may comprise, or be based upon, a passport copy, a passport number, a birth certificate number, or a national insurance number. For example, the identifier may comprise user biometric data. For example, the identifier may have been previously stored by the user using codes, passwords or other data that are unique to the user.

The authentication component may be configured to provide an indication that the stored data is genuine or authorised. For example, the identifier may be stored in conjunction with a code or encryption or symbol that indicates that the identifier has been uploaded to the authentication component by an authorised person and or via an authorised means such as an official or accredited application.

The identifier may comprise information regarding the user's identity. The identifier may comprise information regarding the user's age. The identifier may comprise information regarding the user's authority to perform a task, for example to make a payment from a particular bank account or from a linked credit card. The identifier may comprise information regarding the user's membership of a particular club, group, institution, place of employment or business, that confers upon the user certain rights or authorities. The identifier may comprise information regarding the user's qualifications or permissions. The identifier may comprise information regarding the user's previous interaction with the authentication requesting device or with another linked authentication requesting device, for example to show that he or she is an existing customer of a retailer or has been granted permission previously to a particular location. The identifier may comprise information regarding the user's previous actions - for example it may comprise information confirming that the user has previously paid for a product or service.

The authentication requesting device may be configured to refuse to authorise performance of the user action if the received identifier indicates that a user does not fulfil at least one predetermined criterion. For example, it may reject the request if the user's age is not equal to or above a predetermined age limit, or if the identifier does not match with a predefined identifier, to which the authentication requesting device has access. For example, the request may be rejected if the user's name or other identifying data does not match with a list of names or other identifying data that is stored on, or accessible to, the authentication requesting device.

The authentication component may comprise a communication interface configured to communicate with the authentication requesting device. The communication interface may be separate from or use a different communication channel or modality from a wireless communication link to a mobile device that is used to control operation of the smoked substitute device. For example, in addition to a communication interface in the authentication component, the smoking substitute device may further comprise a wireless interface configured to wirelessly communicate with an application installed on a mobile device. The wireless interface may comprise any suitable type of wireless communication interface, or terminal, for example a Wi-Fi or Bluetooth™ or Bluetooth™ Low Energy (BLE) interface.

The communication interface of the authentication component may comprise a wireless or contactless interface. For example, the authentication requesting device and the smoking substitute may be configured to communicate with one another using Near-Field Communication (NFC). The smoking substitute device may comprise, as or in its authentication component, an NFC transceiver or an NFC tag. The NFC tag may act both as a data store and as a contactless interface.

As the skilled reader will know, NFC (Near Field Communication) is a communication technology that relies on physical proximity between two NFC-enabled devices, in order to exchange data between them. In broad terms; NFC employs electromagnetic induction between two loop antennas, which are comprised within the respective devices, when two NFC-enabled devices come to within a predetermined distance of one another.

An NFC transceiver is typically configured to both transmit and receive NFC data. On the other hand, an NFC tag is typically a read-only device that cannot read data from other devices, or request data or receive data, but an NFC tag can transmit data to an NFC-enabled device that has reading capabilities. It is known for an NFC tag to be registerable to a user, for example via a web-based process, in order to store a unique identifier on the NFC tag. As a result, the NFC tag (and any device in which it is comprised) may subsequently be used as means by which the user's identity (or authority or right of access) can be authenticated by a requesting NFC-enabled device.

The smoking substitute device may comprise, as its authentication component, an RFID transceiver or an RFID tag. As the skilled reader will know, RFID (Radio Frequency Identification) is another proximity-based communication technology, that uses electromagnetic interaction between two RFID-enabled devices. Both RFID and NFC communications operate regardless of whether the two enabled devices are in line of sight with one another. Therefore, an NFC tag or an RFID tag can be incorporated physically within a smoking substitute device, not visible from the outside, and will still enable communication.

Moreover, both NFC tags and RFID tags can be provided in physically compact form, and quite inexpensively, and thus can be incorporated into a relatively small device such as a smoking substitute device, without adding significantly to the device's overall size, weight, bulk or financial cost.

The authentication requesting device may be configured to reject the request for performance of the user action if the received identifier indicates that a user does not fulfil at least one predetermined criterion.

The authentication requesting device may have a communications interface of the same type as the communication interface of the smoking substitute device. For example, the two devices may be configured to communicate with one another using Near-Field Communication (NFC) or RFID (Radio Frequency Identification).

The authentication requesting device may comprise any of, for example: a contactless payment terminal, an electronic keypad or electronic reader, configured for unlocking a door or access point, or a device that is configured to obtain electronic signatures. The authentication requesting device may be configured to issue a human-readable signal and/or a computer-readable signal and/or a machine-readable signal, to indicate that it has authorised performance of a user action, upon receipt of the identifier from the smoking substitute device. For example, it may generate a message, bar code, password, URL or other data, that a human user or a computer or machine can use, to proceed with the requested action. The authentication requesting device may be configured to perform an action to indicate that it has authorised performance of a user action, upon receipt of the identifier from the smoking substitute device. For example, it may issue an electronic signal, to unlock a door or other access point. The authenticating requesting device may be configured to output a visible or audible signal, to convey whether or not it has authorised performance of a user action. For example, it may comprise a screen that shows a green 'tick' when a user action has been authorised or a red 'cross' when it has not been authorised.

The user action, for which authorisation is being sought, may be a commercial transaction. The authentication request may comprise a request to authenticate the user, to authorise the performance of a commercial transaction by the user. For example, the authentication request may comprise a request to authenticate the user, to authorise him or her to make an online purchase, or to pay for goods in person, via a bank account or a credit card or a payment website or application that is linked with the smoking substitute device. The identifier may, in some cases, perform a dual task of, for example, verifying the user's age to confirm that it is permissible or legal for him or her to make the transaction and verifying that the user is authorised to instruct a payment from the linked bank account or credit card or payment website or payment application.

The user action, for which authorisation is being sought, may be to obtain physical access to a specified location and the authentication request may comprise a request to authenticate the user, to authorise him or her to obtain physical access the specified location. For example, the user action may be to unlock a door, to permit access to an exclusive or restricted area, such as a lounge that is designated for use only by particular individuals or by members of a particular club or other group. The user action may be to unlock the door that provides access to that area. The authentication device may be configured to instruct the door to unlock if the received identifier, from the smoking substitute device, matches a identifier entry in a predetermined list of identifiers. For example, the identifier may comprise the user's name or membership number or membership code and the door may only be unlocked if the provided user's name or membership number or membership code matches one found on a pre-stored list or file.

The user action, for which authorisation is being sought, may be to provide a signature and the authentication request may comprise a request to authenticate the user, to authorise him or her to provide a signature. For example, a signature may be required to authorise receipt of a delivery. The identifier provided by the smoking substitute device may be provided, in place of the user physically signing for the delivery, for example using an electronic pen and touch screen receiver. The authentication device may be configured to authenticate the user and accept the identifier in lieu of a signature, if the received identifier matches a identifier entry in a predetermined list of identifiers.

According to another aspect, the invention may provide a user authentication method comprising: communicating an authentication request from an authentication requesting device to a smoking substitute device; receiving the authentication request at the smoking substitute device; in response to the received authentication request, communicating a identifier from the smoking substitute device to the authentication requesting device; receiving the identifier at the authentication requesting device; authenticating, by the authentication requesting device, the user using the identifier; and authorising, by the authentication requesting device, a user action if the user is successfully authenticated. The method may be performed by the system discussed above.

According to another aspect, the invention may provide authentication method comprising: receiving, at a smoking substitute device, an authentication request from an authentication requesting device; and in response to the received authentication request, communicating, by an authentication component on the smoking substituted device, a identifier from the smoking substitute device to the authentication requesting device.

According to another aspect, the invention may provide a computer-readable medium containing instructions configured to, when executed by a processor, perform any method disclosed herein.

According to another aspect, the invention may provide a smoking substitute device comprising an authentication component configured for communication with an authentication requesting device. The smoking substitute device is configured to: receive an authentication request from an authentication requesting device; and in response to the received authentication request, communicate an identifier to the authentication requesting device. As discussed above, the identifier may be suitable for use, by the authentication requesting device, to authenticate the user to authorise performance of the user action.

The authentication component may include a passive communication tag that on which the identifier is encoded. For example, the passive communication tag may be an NFC tag or an RFID tag. The smoking substitute device may thus operate as a contactless authentication unit. The unit may be energised via magnetic or electromagnetic means, for example based on proximity to an authentication requesting device.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### Summary of the Figures

Embodiments and experiments illustrating the principles of the invention will now be discussed with reference to the accompanying figures in which:
**Figure 1** shows an example system for managing a smoking substitute device.
**Figure 2(a)** shows an example smoking substitute device for use as the smoking substitute device in the system of Fig. 1.
**Figure 2(b)** shows the main body of the smoking substitute device of Fig. 2(a) without the consumable.
**Figure 2(c)** shows the consumable of the smoking substitute device of Fig. 2(a) without the main body.
**Figure 3(a)** is a schematic view of the main body of the smoking substitute device of Fig. 2(a), according to an embodiment of the invention.
**Figure 3(b)** is a schematic view of the consumable of the smoking substitute device of Fig. 2(a).
**Figure 4** is a flow diagram of operations carried out by a smoking substitute device and an authentication requesting device, according to an embodiment of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

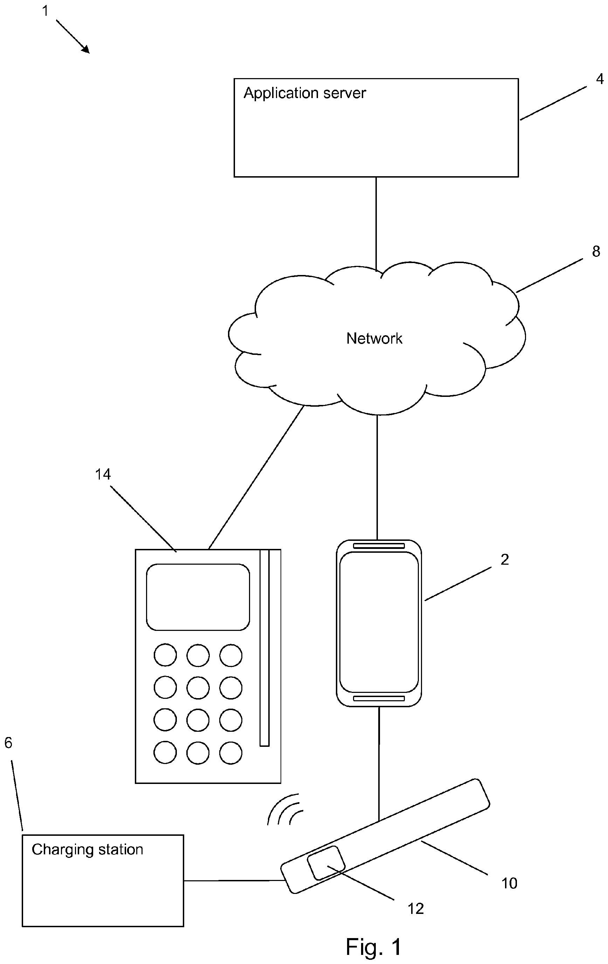

Fig. 1 shows an example system 1 for managing a smoking substitute device 10.

The system 1 as shown in Fig. 1 includes a mobile device 2, an application server 4, an optional charging station 6, as well as the smoking substitute device 10. The system 1 further comprises an authentication requesting device 14, which in this example is a contactless payment terminal configured to obtain authentication information, e.g. from bank cards or suitably configured mobile devices, in order to authorise and process payment. The authentication requesting device 14 may be in communication via the network 8 with the application server 4 or another authorising entity capable of verifying identity information provided thereto.

In this example, the smoking substitute device 10 includes an authentication component 12 that is arranged to provide identification information about the user in response to an authentication request. The authentication component 12 may operate wirelessly over a short range. In particular, the authentication component 12 may be a passive component that is operable when in proximity to a suitable transceiver. The authentication component 12 may include a near field communication (NFC) tag or a radiofrequency identification (RFID) tag encoded with information that is indicative of the user's identity.

The smoking substitute device 10 is configured to communicate wirelessly, e.g. via Bluetooth™, with an application (or "app") installed on the mobile device 2, e.g. via a suitable wireless interface (not shown) on the mobile device 2. The mobile device 2 may be a mobile phone, for example. The application on the mobile phone is configured to communicate with the application server 4, via a network 8. The application server 4 may utilise cloud storage, for example.

The network 8 may include a cellular network and/or the internet.

A skilled person would readily appreciate that the mobile device 2 may be configured to communicate via the network 8 according to various communication channels, preferably a wireless communication channel such as via a cellular network (e.g. according to a standard protocol, such as 3G or 4G) or via a WiFi network.

The app installed on the mobile device and the application server 4 may be configured to assist a user with their smoking substitute device 10, based on information communicated between the smoking substitute device 10 and the app and/or information communicated between the app and the application server 4.

The charging station 6 (if present) may be configured to charge (and optionally communicate with) the smoking substitute device 10, via a charging port on the smoking substitute device 10. The charging port on the smoking substitute device 10 may be a USB port, for example, which may allow the smoking substitute device to be charged by any USB-compatible device capable of delivering power to the smoking substitute device 10 via a suitable USB cable (in this case the USB-compatible device would be acting as the charging station 6). Alternatively, the charging station could be a docking station specifically configured to dock with the smoking substitute device 10 and charge the smoking substitute device 10 via the charging port on the smoking substitute device 10.

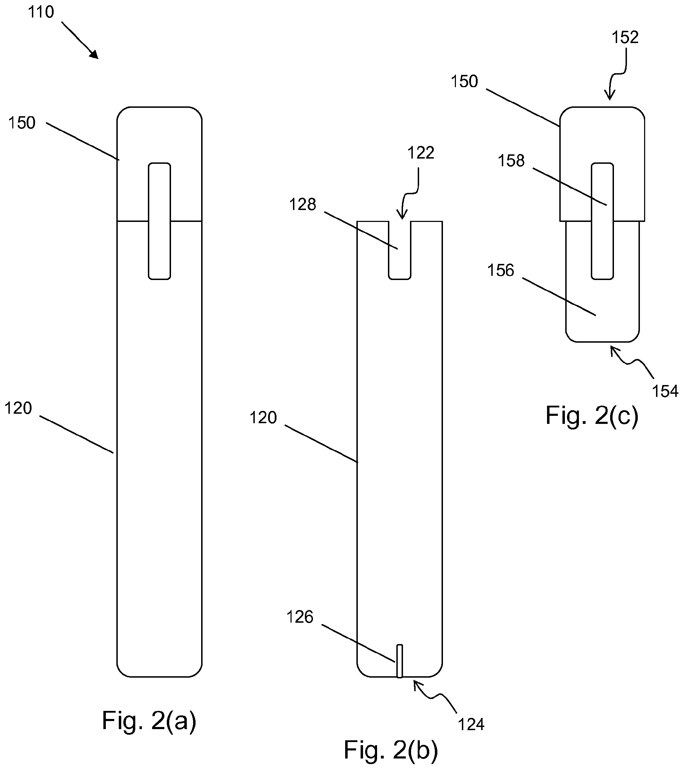

Fig. 2(a) shows an example smoking substitute device 110 for use as the smoking substitute device 10 in the system 1 of Fig. 1.

In this example, the smoking substitute device 110 includes a main body 120 and a consumable 150. The consumable 150 may alternatively be referred to as a "pod".

In this example, the smoking substitute device 110 is a closed system vaping device, wherein the consumable 150 includes a sealed tank 156 and is intended for one-use only.

Fig. 2(a) shows the smoking substitute device 110 with the main body 120 physically coupled to the consumable 150.

Fig. 2(b) shows the main body 120 of the smoking substitute device 110 without the consumable 150.

Fig. 2(c) shows the consumable 150 of the smoking substitute device 110 without the main body 120.

The main body 120 and the consumable 150 are configured to be physically coupled together, in this example by pushing the consumable 150 into an aperture in a top end 122 of the main body 120, e.g. with the consumable 150 being retained in the aperture via an interference fit. In other examples, the main body 120 and the consumable could be physically coupled together by screwing one onto the other, through a bayonet fitting, or through a snap engagement mechanism, for example. An optional light 126, e.g. an LED located behind a small translucent cover, is located a bottom end 124 of the main body 120. The light 126 may be configured to illuminate when the smoking substitute device 110 is activated.

The consumable 150 includes a mouthpiece (not shown) at a top end 152 of the consumable 150, as well as one or more air inlets (not shown in Fig. 2) so that air can be drawn into the smoking substitute device 110 when a user inhales through the mouthpiece. At a bottom end 154 of the consumable 150, there is located a tank 156 that contains e-liquid. The tank 156 may be a translucent body, for example.

The tank 156 preferably includes a window 158, so that the amount of e-liquid in the tank 156 can be visually assessed. The main body 120 includes a slot 128 so that the window 158 of the consumable 150 can be seen whilst the rest of the tank 156 is obscured from view when the consumable 150 is inserted into the aperture in the top end 122 of the main body 120.

In this present embodiment, the consumable 150 is a "single-use" consumable. That is, upon exhausting the e-liquid in the tank 156, the intention is that the user disposes of the whole consumable 150. In other embodiments, the e-liquid (i.e. aerosol former) may be the only part of the system that is truly "single-use". In such embodiments, the tank 156 may be refillable with e-liquid or the e-liquid may be stored in a non-consumable component of the system. For example, the e-liquid may be stored in a tank located in the device or stored in another component that is itself not single-use (e.g. a refillable tank).

The tank 156 may be referred to as a "clearomizer" if it includes a window 158, or a "cartomizer" if it does not.

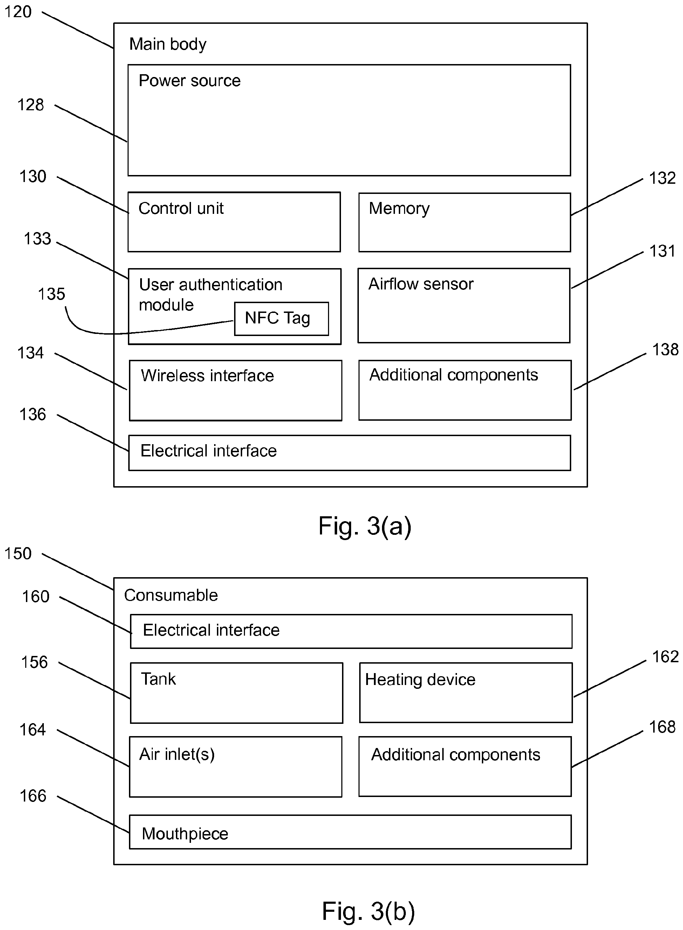

Fig. 3(a) is a schematic view of the main body 120 of the smoking substitute device 110.

Fig. 3(b) is a schematic view of the consumable 150 of the smoking substitute device 110.

As shown in Fig. 3(a), the main body 120 includes a power source 128, a control unit 130, an airflow sensor 131, a memory 132, a user authentication module 133, a wireless interface 134, an electrical interface 136, and, optionally, one or more additional components 138.

The power source 128 is preferably a battery, more preferably a rechargeable battery.

The control unit 130 may include a microprocessor, for example.

The memory 132 is preferably includes non-volatile memory.

The wireless interface 134 is preferably configured to communicate wirelessly with the mobile device 2, e.g. via Bluetooth. To this end, the wireless interface 134 could include a Bluetooth™ antenna. Other wireless communication interfaces, e.g. WiFi, are also possible.

The electrical interface 136 of the main body 120 may include one or more electrical contacts. The electrical interface 136 may be located in, and preferably at the bottom of, the aperture in the top end 122 of the main body 120. When the main body 120 is physically coupled to the consumable 150, the electrical interface 136 may be configured to pass electrical power from the power source 128 to (e.g. a heating device of) the consumable 150 when the smoking substitute device 110 is activated, e.g. via the electrical interface 160 of the consumable 150 (discussed below). When the main body 120 is not physically coupled to the consumable 150, the electrical interface may be configured to receive power from the charging station 6.

The additional components 138 of the main body 120 may include the optional light 126 discussed above.

The additional components 138 of the main body 120 may, if the power source 128 is a rechargeable battery, include a charging port configured to receive power from the charging station 6. This may be located at the bottom end 124 of the main body 120. Alternatively, the electrical interface 136 discussed above is configured to act as a charging port configured to receive power from the charging station 6 such that a separate charging port is not required.

The additional components 138 of the main body 120 may, if the power source 128 is a rechargeable battery, include a battery charging control circuit, for controlling the charging of the rechargeable battery. However, a battery charging control circuit could equally be located in the charging station 6 (if present).

The additional components 138 of the main body 120 may include an airflow sensor for detecting airflow in the smoking substitute device 110, e.g. caused by a user inhaling through a mouthpiece 166 (discussed below) of the smoking substitute device 110. The smoking substitute device 110 may be configured to be activated when airflow is detected by the airflow sensor. This optional sensor could alternatively be included in the consumable 150 (though this is less preferred where the consumable 150 is intended to be disposed of after use, as in this example).

The airflow sensor 131 is configured to detecting airflow in the smoking substitute device 110, e.g. caused by a user inhaling through a mouthpiece 166 (discussed below) of the smoking substitute device 110. The smoking substitute device 110 may be configured to be activated when airflow is detected by the airflow sensor.

The additional components 138 of the main body 120 may include an actuator, e.g. a button. The smoking substitute device 110 may be configured to be activated when the actuator is actuated. This provides an alternative to the airflow sensor noted, as a mechanism for activating the smoking substitute device 110.

The additional components 138 of the main body 120 may include a reader configured to read information associated with the consumable from a machine readable data source included in (e.g. contained in the body of, or attached to) the consumable 150.

The reader may be configured to read information from the machine readable data source wirelessly, e.g. via electromagnetic waves or optically. Thus, for example, the machine readable data source included in the consumable 150 could be an RFID tag (in which case the reader included in the main body 120 may be an RFID reader) or a visual data source such as a barcode (in which case the reader included in the main body may be an optical reader, e.g. a barcode scanner). Various wireless technologies and protocols may be employed to allow the reader to wirelessly read information from a machine readable data source included in or attached to the consumable 150, e.g. NFC, Bluetooth, Wi-Fi, as would be appreciated by a skilled person.

For avoidance of any doubt, the reader (if present) may be configured to read information from the machine readable data source non-wirelessly, e.g. using a direct electrical connection between the main body 120 and consumable 150.

As shown in Fig. 3(b), the consumable 150 includes the tank 156, an electrical interface 160, a heating device 162, one or more air inlets 164, a mouthpiece 166, and, optionally, one or more additional components 168.

The electrical interface 160 of the consumable 150 may include one or more electrical contacts. The electrical interface 136 of the main body 120 and an electrical interface 160 of the consumable 150 are preferably configured to contact each other and therefore electrically couple the main body 120 to the consumable 150 when the main body 120 is physically coupled to the consumable 150. In this way, electrical energy (e.g. in the form of an electrical current) is able to be supplied from the power source 128 in the main body 120 to the heating device 162 in the consumable 150.

The heating device 162 is preferably configured to heat e-liquid contained in the tank 156, e.g. using electrical energy supplied from the power source 128. In one example, the heating device 162 may include a heating filament and a wick, wherein a first portion of the wick extends into the tank 156 in order to draw e-liquid out from the tank 156, and wherein the heating filament coils around a second portion of the wick located outside the tank 156. In this example, the heating filament is configured to heat up e-liquid drawn out of the tank 156 by the wick to produce an aerosol vapour.

The one or more air inlets 164 are preferably configured to allow air to be drawn into the smoking substitute device 110, when a user inhales through the mouthpiece 166.

The additional components 168 of the consumable 150 may include a machine readable data source, which may e.g. be contained in the body of, or attached to the consumable 150. The machine readable data source may store information associated with the consumable. The information associated with the consumable may include information concerning the content of the consumable (e.g. e-liquid type, batch number) and/or a unique identifier, for example.

The machine readable data source may be rewritable, e.g. a rewritable RFID chip, or read only, e.g. a visual data source such as a barcode. As indicated above, the additional components 138 of the main body 120 may include a reader configured to read information associated with the consumable from the machine readable data source.

In use, a user activates the smoking substitute device 110, e.g. through actuating an actuator included in the main body 120 or by inhaling through the mouthpiece 166 as described above. Upon activation, the control unit 130 may supply electrical energy from the power source 128 to the heating device 162 (via electrical interfaces 136, 166), which may cause the heating device 162 to heat e-liquid drawn from the tank 156 to produce a vapour which is inhaled by a user through the mouthpiece 166.

Of course, a skilled reader would readily appreciate that the smoking substitute device 110 shown in Figs. 2 and 3 shows just one example implementation of a smoking substitute device, and that other forms of smoking substitute device could be used as the smoking substitute device 10 of Fig. 1.

By way of example, a HNB smoking substitute device including a main body and a consumable could be used as the smoking substitute device 10 of Fig. 1, instead of the smoking substitute device 110. One such HNB smoking substitute device is the IQOS™ smoking substitute device discussed above.

As another example, an open system vaping device which includes a main body, a refillable tank, and a mouthpiece could be used as the smoking substitute device 10 of Fig. 1, instead of the smoking substitute device 110. One such open system vaping device is the blu PRO™ e-cigarette discussed above.

As another example, an entirely disposable (one use) smoking substitute device could be used as the smoking substitute device 10 of Fig. 1, instead of the smoking substitute device 110.

Embodiments of the present invention relate to use of a smoking substitute device for functions beyond its core function of enabling smoking substitute action. In particular, they relate to using a smoking substitute device to provide a identifier, that enables the user to be authenticated by an authentication requesting device, and thereby enables the user to be authorised to carry out or continue a particular action and/or to access a particular location and/or to take receipt of a particular item. The invention may permit the smoking substitute device to be used alone to authenticate the identity of the user. In other words, in the invention it need not be necessary to rely on knowledge of any other device that may be associated with the smoking substitute device, such as the mobile device 2 discussed above. All information needed to authenticate the user may be stored on and communicable by the smoking substitute device directly to an authentication requesting device.

The invention relates to various types of smoking substitute device, including those mentioned above.

In embodiments of the invention, the smoking substitute device 110 comprises a user authentication module 133. The user authentication module 133 is configured to permit the smoking substitute device 110 to be used as a means for confirming the identity or other information about the user, e.g. a registered user of the smoking substitute device. The user authentication module 133 may be arranged to operate in a contactless manner. For example, the user authentication module 133 may include an NFC (Near Field Communication) tag 135, that may be embedded within the main body 120 of the smoking substitute device 110. The user authentication module 133 may use other components for contactless or wireless transmission of identity information, e.g. an RFID tag or the like.

As the skilled reader will be aware, in general terms, NFC is a standard for very short-range radio transmission. NFC is similar to RFID but tends to have a much shorter physical range. This shorter range is not a problem for the implementations discussed herein - and in fact is an advantage for implementations such as contactless payment and unlocking of electronic locks, because the short range requires the user and his or her smoking substitute device 110 to be physically very close to the reader (i.e. to the authentication requesting device), and therefore it reduces the risk of the wrong NFC tag being read, in any particular situation. Therefore, it reduces the risk of fraud or other potential misuse.

Both NFC tags and RFIDS tags comprise a (relatively low capacity) memory, and a radio chip, in communication with an antenna. NFC tags such as the NFC tag 135 in this embodiment are passive, and do not include a power source. Instead, the NFC tag 135 is configured to draw power from an NFC-enabled device that reads it, through a process of magnetic induction. When an NFC-enabled reader comes within sufficiently close proximity to the tag 135, it will energise the tag 135 and enable transfer data from the tag 135 to the reader.

The user authentication module may be configured as an electronic identification card, and may be configured to comply with any relevant standard, e.g. ISO/IEC 7816. Other types of NFC tag may be used, but the NFC tag 135 should be of a reliably secure standard.

The user authentication module 133 may be interrogated by any suitable authentication requesting device. For example, the authentication requesting device may be a contactless payment terminal or an electronic door release or an age verification means, configured to permit certain user actions only if the user is of a predefined age (or older). It may be comprised within an electronic recordal device, for recording receipt of delivered items.

The authentication requesting device may comprise an NFC transceiver, which is configured to read NFC tags such as the NFC tag 135 in the smoking substitute device 110. It may also include a display, on which an output indicating authentication confirmation (or refusal) may be output. In this embodiment, For example, the display may be configured to show a green 'tick' when a user action has been authorised or a red 'cross' when it has not been authorised. The authentication requesting device ,may additional components, for example a memory, a control unit and a power source, but these are not discussed in detail herein. The authentication requesting device may be network enabled, for example comprising a Wi-Fi, 4G or Bluetooth™ interface, in order for it to access information stored on a server, website, or application. The authentication requesting device may comprise any suitable hardware and software means, in order to enable it to function as described herein.

The authentication requesting device in this embodiment may be comprised within, or linked to, another device or computer that is configured to perform or facilitate user actions. For example, it may be comprised within a 'cash register' or payment receipt computer, wherein an authentication output from the authentication requesting device determines whether a user's payment attempt, via his or her smoking substitute device 110, will be allowed to proceed.

In this embodiment, after first purchase of the smoking substitute device 110, the user can register the NFC tag 135, via a suitable web-based or application-based process. For example, the user may input information identifying their particular smoking substitute device with details relating to their identity, age, banking information via a secure website that is in communication with the application server 4 or another suitable server at the back end of the system 1. The smoking substitute device may include a serial number, bar code or other identification information. The back end of the system 1 may associate the (public) identification information of the smoking substitute device with a (private) identifier of the NFC tag 135. Upon receiving an authentication request, the NFC tag 135 may output its identifier, e.g. in encrypted form. The authentication requesting device may use the identifier to authenticate the user, e.g. by communicating with the back end to verify that the identifier is associated with user information that permits a certain action to be taken. In other examples, the identifier may be used to authorize other actions, e.g. contactless payment.

It is known for NFC tags to be customisable by an end user. In this example, a registration procedure may be performed to encode identity information into the NFC tag. For example, the NFC tag may include a flag that can be set to indicate that a user has completed an age verification procedure. In this example, the smoking substitute device itself can be used to confirm a user's age, which necessarily requiring communication to a back end system.

In one embodiment, the user can use an application running on a mobile device - for example, a mobile phone, smartphone tablet, or laptop computer - in order to record one or more unique identifiers on, or in association with, that individual NFC tag 135, wherein the application or a server may also securely store the one or more unique identifiers, so that they can be used as means by which the user's identity can be subsequently authenticated by an NFC-enabled requesting device. The process of recording the one or more unique identifiers should be secure, for example involving the exchange of encryption keys or other security codes, to ensure that the integrity of the data is maintained and to prevent misuse by non-authorised users. For example, the unique identifiers may be encrypted at a back end server, so that the mobile device cannot access the information therein.

In one example, the user may use an online banking application in order to register or otherwise link the NFC tag 135 to a bank account. The registration may include associating (public) identification information of the smoking substitute device with a (private) identifier of the NFC tag, as discussed above. It is known for a user to use a number of user-specific security means (for example codes, passwords, pre-stored answers to security questions, recognition of pre-stored images or fingerprint data) in order to securely access an online banking application on a mobile device, for example on his or her smartphone. According to this embodiment, when the user has securely accessed to his or her online banking application, he or she can link the NFC tag 135 to his or her bank account via a number of different methods, as detailed below.

If the mobile device on which the online banking application is installed itself has an NFC transceiver, then the user can activate the NFC capabilities of the mobile device, via the online banking application, bring the mobile device into close proximity with the smoking substitute device 110 in which the NFC tag 135 is comprised, and provide any further confirmation that the application demands in order to link that NFC tag 135 to the user's bank account. In response to the user confirming that the NFC tag 135 should be linked to the user's bank account, the NFC transceiver within the mobile device will use Near Field Communication to transfer secure account-specific data to the NFC tag 135, for storage thereon. The data should be stored in an encrypted manner, to prevent access to it by non-authorised persons. The stored secure account-specific data on the NFC tag 135 will enable it to be subsequently used in a similar manner to a contactless debit card, in that a suitable NFC-reader such as a retail payment terminal will be able to read the data, and from it ascertain the user's payment information. It will also be able to ascertain that the data has been securely stored, by the authorised user, so that the payment terminal can authenticate the user and authorise the payment.

An alternative method for registering the NFC tag 135 to a user's bank account is to use a unique serial number or other secure identifier for the NFC tag 135 - which would be supplied by the manufacturer or supplier of the smoking substitute device 110, on purchase - and to register that serial number to the user's bank account, via the online banking application. If, subsequently, the NFC tag 135 (within the smoking substitute device 110) is brought into close proximity with a suitable NFC-reader, such as a retail payment terminal, the reader will be able to read the unique serial number and access a suitable database to check if it has been registered to a specific user or bank account, and from there, ascertain the user's payment information. It will also be able to ascertain that the data has been securely stored, by the authorised user, so that the payment terminal can authenticate the user and authorise the payment.

The user-specific data that is written to the tag 135 may comprise a URL or other link to an application or other database, on which one or more identifiers have been stored. Alternatively, actual user identification data may be stored locally on the NFC tag itself. Any locally stored data and any links should be stored in an encrypted form, or in another secure form to prevent potential misuse. For example, the user's name, a membership number for a particular club or association, passport information or banking information, may be stored in an encrypted form that can only be read by authorised NFC-enabled authentication requesting devices - such as contactless payment terminals, NFC-enabled electronic locks and so on.

The user identification data may include data that verifies the user's age - for example it may comprise passport information, or other age-related data, input by the user. According to an embodiment, when an authentication requesting device reads the NFC tag 135, it may derive both age data and payment data from the tag 135, in order to ascertain both that the user is of a permissible age to make the purchase and to obtain the banking/payment details for the transaction.

The identifier may be stored with a particular code or encryption or symbol that indicates that the identifier has been uploaded to the tag 135 by an authorised person and/or via an authorised means such as an official or accredited application.

Although an exemplary embodiment has been described herein, which makes use of an online banking application, for providing identifiers that relate to payment authorisation, it will be appreciated that other types of application, and other types of identifiers, may be made use of, according to the present invention. For example, a user may store identifiers on his or her NFC tag via a specific NFC tag customisation application, running on a mobile device, wherein those identifiers may subsequently be readable by more than one type of authentication requesting device, and/or may be used to authorise more than one type of user action. For example, a user may store identifiers on his or her NFC tag via an application that relates to a particular location, in order to grant the user access to that location, by using his or her NFC tag.

In an embodiment, the user may register his or her NFC tag, and upload identifier data to it, by instructing the manufacturer or developer of the smoking substitute device 110 to customise the NFC tag 135 for that user. This may be done by the manufacturer or developer issuing a user-specific firmware update, for the user to download via an application on a linked mobile device, or the manufacturer or developer may need physical access to the smoking substitute device, to make the customisation.

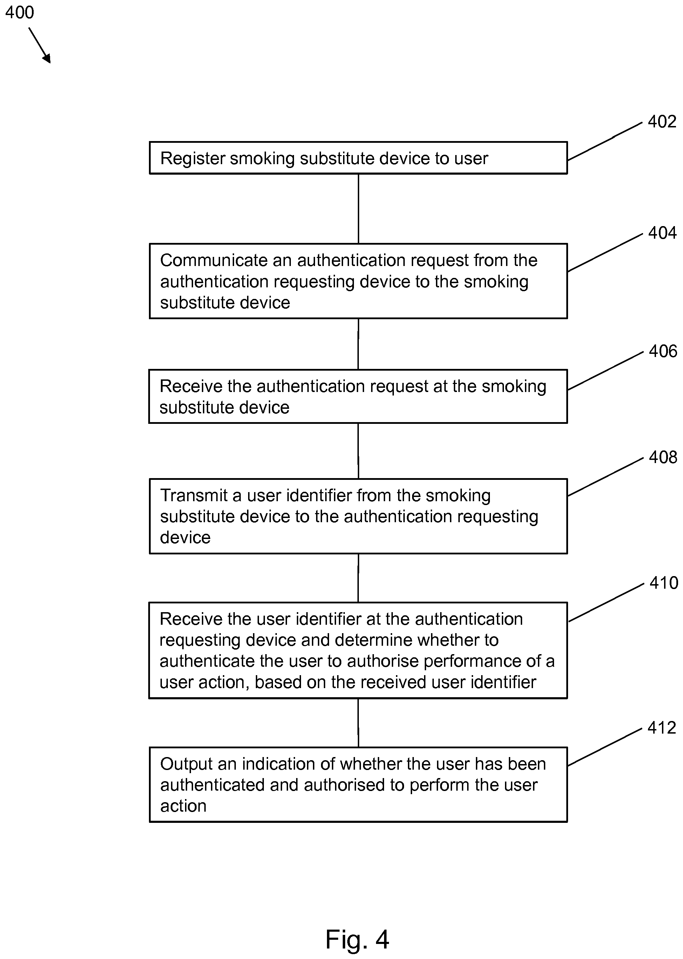

Fig. 4 shows the steps followed by the smoking substitute device 110 and the authentication requesting device in a method that is an embodiment of the present invention.

Step 402 comprises registering the smoking substitute device to a user. This step may involve linking the user with an output produced by the user authentication module, e.g. in the manner discussed above.

Once the user authentication module in the smoking substitute 110 is set up, the smoking substitute device may be ready to authenticate a user when the user requests or attempts to perform a certain user action. The request or attempt may simply comprise the user bringing the smoking substitute device into close proximity with the authentication requesting device, e.g. in response to a request the authentication requesting device that authentication data is required. For example, when the authentication requesting device comprises a payment terminal, the user bringing the smoking substitute device into close proximity with the authentication requesting device can be taken as a request by the user to obtain authentication in order to make a payment. When the authentication requesting device is an electronic door release, the user bringing the smoking substitute device into close proximity with the authentication requesting device can be taken as a request by the user to obtain authentication in order access the area or location that the door provides access to. In other cases, the nature of the user action for which authorisation is being sought will not be apparent or confirmed to the authentication requesting device merely by the smoking substitute device coming into close proximity with it.

At step 404 the authentication requesting device communicates an authentication request to the smoking substitute device. This request may be transmitted by a NFC transceiver to the NFC tag 135. As discussed above, by bringing the two devices into close proximity (e.g. within 5 cm or 10 cm) of one another, a magnetic induction loop will be formed between the transceiver and the tag 135. This magnetic induction loop has the effect of conveying energy to the NFC tag 135 and thereby enabling it to receive the authentication request, at step 406.

At step 408 the NFC tag 135 responds to the authentication request by transmitting an identifier to the NFC transceiver. As detailed above, the identifier may be pre-stored on the NFC tag 135 or may be written thereon as part of a registration process.

At step 410 the authentication requesting device receive the identifier and makes a determination regarding whether or not to authenticate the user to authorise performance of a user action. As detailed above, this process may involve an interim step whereby the authentication requesting device uses the information that it has received from the NFC tag 135 in order to access an application website or other database in order to obtain corresponding user identifying information. Such an interim step requires the authentication requesting device to be network enabled. In order to make a determination whether or not to authenticate the user, the authentication requesting device must determine whether the identifier that it has received meet a pre-determined criterion. For example, it may check whether the received identifier corresponds to a pre-stored identifier on a database. Alternatively, or additionally it may check whether the identifier includes a code or encryption or symbol that indicates that the identifier has been uploaded to the tag 135 by an authorised person and/or via an authorised means such as an official or accredited application. In another example, the identifier itself may be encoded with information that identifies the user or communicates certain information about them, e.g. age or the like. In such an example, the authentication requesting device may be able to determine whether or not to authenticate the user (or permit performance of a certain action) without having to communicate with a third party.

At step 412, if it has been determined that the user can be authenticated and thus authorisation can be given for performance of a user action, the authentication requesting device can output an indication accordingly. For example, the indication can be output on a visual display and/or as an audio signal. In addition, the authentication requesting device may be configured to output a command either to other components within the authentication requesting device itself and/or to components of a linked device or computer, to instruct it to carry out a particular action. For example, the particular action may be to complete a transaction or to open an electronic lock. For example, the particular action may be to provide an indication to a database such as a delivery database that an authenticated user has, in effect, signed for delivery of (or taken receipt of) an item.

Thus it can be seen that a system method and devices are provided that enable a smoking substitute device to communicate with an authentication requesting device such as a payment terminal, an electronic lock, or a delivery recordal device, in order to provide user authentication and thus to enable the user to perform an action for which he or she requires authorisation. Once the smoking substitute device has been initially configured to store one or more identifiers within an authentication component such as an NFC tag or RFID tag, the smoking substitute device can subsequently operate independently of any other devices in order to provide user authentication. The smoking substitute device does not need to wirelessly communicate with an application on a mobile device or with a server or with any other network entity in order to convey information from its authentication component to an authentication requesting device. However, it may require interaction with a mobile device, application, server or other network entity in order to be initially configured with the requisite identifier data for subsequent use in user authentication processes.

The hardware and software components required in order to put the present invention into effect are not computationally or physically burdensome on the smoking substitute device. They would not require any significant physical change to be made to an existing smoking substitute device configuration, in order to be comprised therewithin. Nor would their inclusion be expensive.

The smoking substitute device provided according to this invention enables the user to perform functions beyond smoking substitute action and thus enhances the potential usefulness of the device to the user. It also provides the potential for the user to carry fewer separate devices on their person in order to carry out day-to-day tasks. And it provides potential for the user to, for example, 'go cashless', which many users find attractive.

The terms authentication component', identifier', authentication requesting device' and so on are intended to be illustrative of the functions performed by those features and need not be limited to a particular device or structure.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A user authentication system comprising:
a smoking substitute device comprising an authentication component; and
an authentication requesting device configured to communicate an authentication request to the smoking substitute device to authorise a user action,
wherein the smoking substitute device is configured to:
receive the authentication request; and
in response to the received authentication request, communicate a identifier to the authentication requesting device,
wherein the authentication requesting device is capable of authenticating the user using the identifier to authorise performance of the user action.

2. The system of claim 1, wherein the authentication requesting device is configured to authorise performance of the user action if the received identifier indicates that a user fulfils at least one predetermined criterion.

3. The system of claim 1 or claim 2, wherein the user action is a commercial transaction and the authentication request comprises a request to authenticate the user in order to authorise the performance of a commercial transaction.

4. The system of any preceding claim, wherein the authentication component comprises a communication interface configured to communicate with the authentication requesting device.

5. The system of claim 4, wherein the communication interface comprises a wireless or contactless interface.

6. The system of claim 5, wherein the authentication requesting device and the smoking substitute are configured to communicate with one another using Near-Field Communication (NFC).

7. The system of any preceding claim, wherein the authentication requesting device is configured to reject the request for performance of the user action if the received identifier indicates that a user does not fulfil at least one predetermined criterion.

8. The system of any preceding claim, wherein the user action is to obtain physical access to a specified location and the authentication request comprises a request to authenticate the user, to obtain physical access the specified location.

9. The system of any preceding claim, wherein the user action is to provide a signature and the authentication request comprises a request to authenticate the user in order to provide a signature.

10. A user authentication method comprising:
communicating an authentication request from an authentication requesting device to a smoking substitute device;
receiving the authentication request at the smoking substitute device;
in response to the received authentication request, communicating a identifier from the smoking substitute device to the authentication requesting device;
receiving the identifier at the authentication requesting device;
authenticating, by the authentication requesting device, the user using the identifier; and
authorising, by the authentication requesting device, a user action if the user is successfully authenticated.

11. A user authentication method comprising:
receiving, at a smoking substitute device, an authentication request from an authentication requesting device; and
in response to the received authentication request, communicating, by an authentication component on the smoking substituted device, a identifier from the smoking substitute device to the authentication requesting device.

12. A computer-readable medium containing instructions configured to, when executed by a processor, perform the method of claim 10 or claim 11.

13. A smoking substitute device comprising:
an authentication component configured for communication with an authentication requesting device, wherein the smoking substitute device is configured to:
receive an authentication request from an authentication requesting device;
in response to the received authentication request, communicate an identifier to the authentication requesting device.

14. The smoking substitute device of claim 13, wherein the authentication component includes a passive communication tag that on which the identifier is encoded.

15. The smoking substitute device of claim 14, wherein the passive communication tag is an NFC tag or an RFID tag.
